# DEMANDE DE BREVET EUROPEEN

(11) **EP 1 291 412 A1**
(43) Date de publication de la demande: **12.03.2003**
(21) Numéro de dépôt: 02292209.0
(22) Date de dépôt: 09.09.2002
(51) Int. Cl.: C12M 1/107, C12M 1/113, C12M 1/40, B01J 19/18

(54) **Réacteur de type à axe horizontal destiné à contenir un effluent liquide pour permettre le développement d'une réaction chimique de celui-ci**

(30) Priorité: 10.09.2001 FR 0111885
(71) Demandeur: Algotec International S.A.R.L., 29710 Ploneis (FR)
(72) Inventeur: Roussel, Jean, 29700 Plomelin (FR)
(74) Mandataire: Maillet, Alain

(57) **Abrégé**

La présente invention concerne un réacteur (100) de type à axe horizontal destiné à contenir un effluent liquide (E) pour permettre le développement d'une réaction chimique de celui-ci, le réacteur (100) étant pourvu d'au moins un dispositif de brassage (200).

Le réacteur (100) est remarquable en ce que le dispositif de brassage (200) comprend un berceau (210) disposé dans le réacteur (100) et sur lequel est monté au moins un support d'accrochage (220) permettant aux bactéries de l'effluent (E) de s'y accrocher, le berceau (210) étant fixé à un moyen d'entraînement (250) permettant au berceau (210) de se déplacer dans un volume pratiquement égal à celui occupé par l'effluent (E).

## Description

La présente invention concerne un réacteur de type à axe horizontal destiné à contenir un effluent liquide pour permettre le développement d'une réaction chimique de celui-ci, en particulier un réacteur de méthanisation.

Dans un tel réacteur, il convient de développer une digestion anaérobie des matières organiques de l'effluent à traiter en les transformant principalement et le plus complètement possible en méthane et en gaz carbonique. L'effluent résiduel peut être valorisé sous forme d'engrais. Outre le contrôle des paramètres comme la température, le pH de l'effluent en cours de digestion, le brassage de l'effluent revêt une importance déterminante pour obtenir un rendement élevé d'un tel réacteur. Il faut en effet multiplier les chances de rencontre entre les micro-organismes avec la matière à dégrader de l'effluent à traiter.

Dans la pratique, on utilise classiquement un ou plusieurs arbres disposés à rotation dans le réacteur et autour desquels sont montés une vis sans fin et/ou des disques s'étendant radialement, comme le suggère le document EP-A-0 203 300.

On peut également utiliser un agitateur pourvu d'un mouvement oscillant, comme le prévoit le document DE-A-31 04 285.

Cependant, ces solutions ne permettent pas de brasser entièrement le volume de l'effluent à traiter et ne permettent également pas à une forte proportion de micro-organismes de participer à la digestion de l'effluent.

Aussi, le but de la présente invention est donc de proposer un réacteur pourvu d'un dispositif de brassage qui permette de brasser la quasi-totalité du volume de l'effluent à traiter et qui puisse également permettre à une forte proportion de micro-organismes de participer à la digestion de l'effluent.

A cet effet, le réacteur de type à axe horizontal est destiné à contenir un effluent liquide pour permettre le développement d'une réaction chimique de celui-ci, le réacteur étant pourvu d'au moins un dispositif de brassage, est remarquable en ce que le dispositif de brassage comprend un berceau disposé dans le réacteur et sur lequel est monté au moins un support d'accrochage permettant aux bactéries de l'effluent de s'y accrocher, le berceau étant fixé à un moyen d'entraînement permettant au berceau de se déplacer dans un volume pratiquement égal à celui occupé par l'effluent.

Ainsi, le dispositif de brassage permet d'atteindre la quasi-totalité du volume de l'effluent et une forte proportion de bactéries participe à la digestion de l'effluent.

Le rendement du réacteur est amélioré par rapport à celui d'un réacteur de l'art antérieur.

Selon une autre caractéristique de l'invention, chaque support d'accrochage est constitué d'un parallélépipède formé à partir de feuilles gaufrées superposées présentant une structure en nids d'abeille.

Selon une autre caractéristique de l'invention, le mouvement du berceau est un mouvement de translation alternatif.

Selon une autre caractéristique de l'invention, la vitesse du berceau en fonctionnement est comprise entre 5 et 50 cm/s.

Selon une autre caractéristique de l'invention, le berceau est constitué d'un cadre fabriqué à partir de profilés métalliques et dans lequel sont fixés le ou les supports d'accrochage.

Selon une autre caractéristique de l'invention, la hauteur du berceau lorsqu'il est installé dans le réacteur est telle qu'il demeure immergé dans l'effluent contenu dans ledit réacteur afin de réduire l'oxygénation de l'effluent à traiter.

Selon une autre caractéristique de l'invention, le moyen d'entraînement est un vérin à double effet.

Selon une autre caractéristique de l'invention, le moyen d'entraînement est un motoréducteur destiné à entraîner en rotation une vis sans fin traversant longitudinalement l'enveloppe et autour duquel au moins un palier taraudé est monté à mouvement hélicoïdal, le palier taraudé étant fixé au berceau.

Selon une autre caractéristique de l'invention, la vis sans fin est de type à double filet croisé permettant un mouvement de translation alternatif du berceau avec un motoréducteur fonctionnant dans un seul sens de rotation.

Selon une autre caractéristique de l'invention, le berceau est suspendu à un dispositif de sustentation destiné à supporter sa masse afin d'éviter que le moyen d'entraînement n'ait à supporter ladite masse et puisse être utilisé uniquement comme moyen de déplacement.

Selon une autre caractéristique de l'invention, le dispositif de sustentation est constitué d'au moins un arbre traversant longitudinalement l'enveloppe et autour duquel au moins un palier est monté coulissant à translation, le palier étant fixé au berceau.

Selon une autre caractéristique de l'invention, le réacteur est un réacteur de méthanisation.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
la Fig. 1 représente une vue en coupe d'un réacteur à axe horizontal selon l'invention,
la Fig. 2a représente une vue de côté d'un berceau destiné à être utilisé comme support à des bactéries selon l'invention,
la Fig. 2b représente une vue de face d'un berceau selon l'invention,
la Fig. 2c représente une vue en coupe d'un berceau selon l'invention, et
la Fig. 3 représente une vue en coupe d'un réacteur dans lequel est représentée une variante de réalisation d'un moyen d'entraînement d'un berceau selon l'invention.

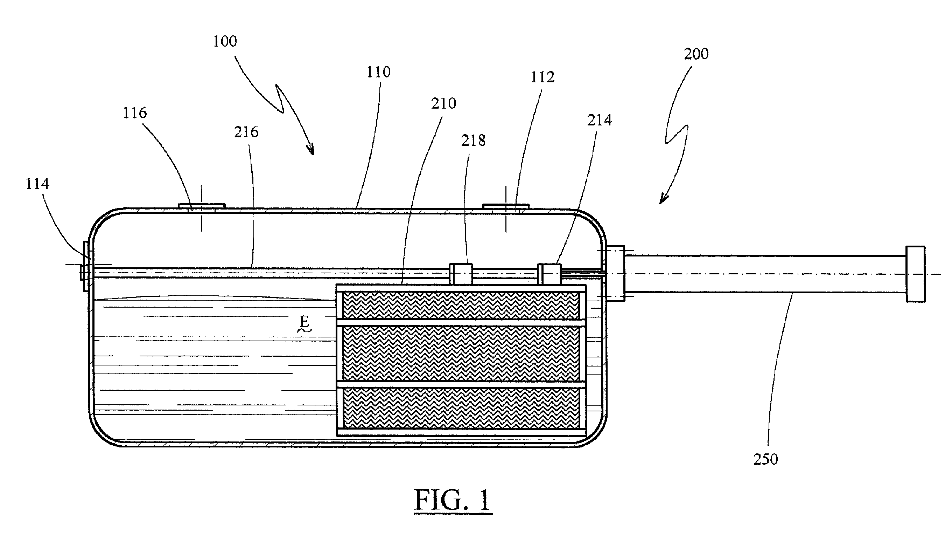

Le réacteur 100 représenté à la Fig. 1 est de type à axe horizontal, c'est-à-dire destiné à être utilisé horizontalement. Un avantage de cette construction est de permettre de rendre plus discret le réacteur en extérieur. Un tel réacteur 100 peut notamment être utilisé comme réacteur de méthanisation par digestion anaérobie d'un effluent E, par exemple un effluent liquide agricole ou industriel.

Ce réacteur 100 est constitué d'une enveloppe 110 destinée à contenir un effluent liquide E. Il est pourvu d'au moins un orifice d'alimentation 112 en effluent et destiné à être raccordé à un circuit d'alimentation en effluent, d'un orifice de dégazage 114 destiné à être raccordé à un circuit de collecte de gaz. L'enveloppe 110 comporte également un orifice de soutirage 116 de l'effluent résiduel.

L'enveloppe 110 peut également comporter un moyen de chauffage tel que des résistances électriques et qui est destiné à chauffer l'effluent E à une température favorisant la digestion anaérobie.

Le réacteur 100 est pourvu d'au moins un dispositif de brassage 200 constitué d'un berceau 210 qui est disposé dans l'enveloppe 110 et qui est pourvu d'au moins un support d'accrochage 220 permettant aux bactéries de l'effluent de s'y accrocher. Le berceau 210 est destiné à être entraîné par un moyen d'entraînement 250.

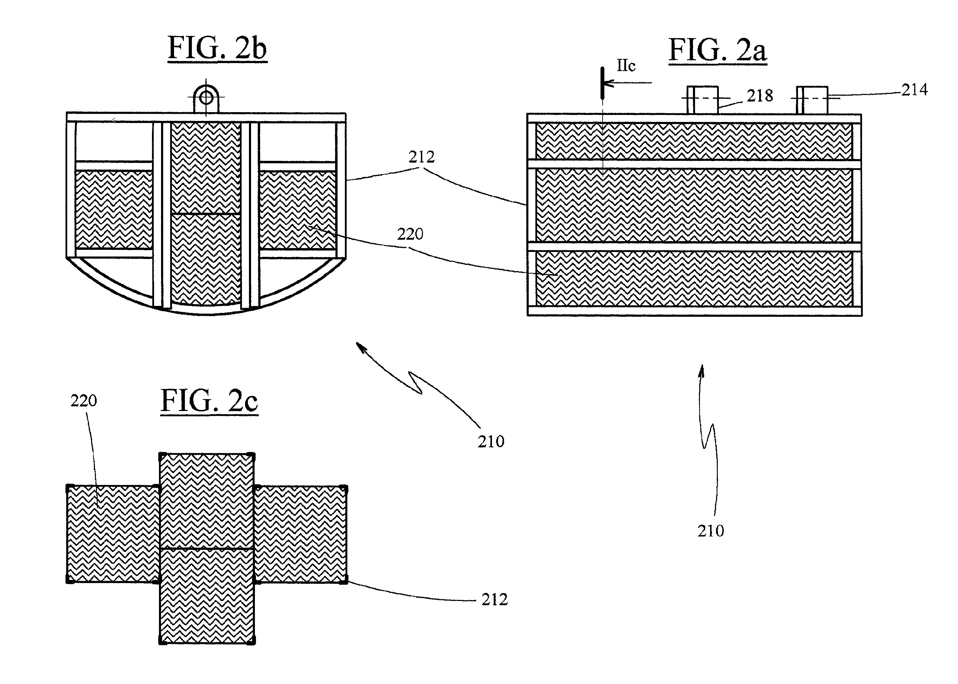

Aux Figs. 2a et 2b, le berceau 210 est constitué d'un cadre fabriqué à partir de profilés métalliques 212 et dans lequel sont fixés le ou les supports d'accrochage 220.

A la Fig. 2c, chaque support d'accrochage 220 est constitué d'un parallélépipède formé à partir de feuilles gaufrées superposées présentant une structure en nids d'abeille. Cette structure particulière présente, au regard de son volume extérieur, une grande densité de surface qui est utilisée comme support d'accrochage pour les bactéries de l'effluent E à traiter.

A la Fig. 1, le berceau 210 peut être entraîné suivant un mouvement de translation alternatif par le moyen d'entraînement 250 constitué d'un vérin à double effet de préférence disposé à l'extérieur du réacteur 100. A cet effet, le berceau 210 est pourvu d'une chape d'entraînement 214 attelée au nez du vérin.

On remarquera que la hauteur du berceau 210 lorsqu'il est installé dans le réacteur 100 est telle qu'il demeure immergé dans l'effluent E contenu dans ledit réacteur 100 afin de réduire l'oxygénation de l'effluent E à traiter.

Le berceau 210 peut être suspendu à un dispositif de sustentation destiné à supporter sa masse afin d'éviter que le moyen d'entraînement 250 n'ait à supporter ladite masse et puisse être utilisé uniquement comme moyen de déplacement. Le dispositif de sustentation représenté à la Fig. 1 est constitué d'au moins un arbre 216 traversant longitudinalement l'enveloppe 110 et autour duquel au moins un palier 218, fixé au berceau 210, est monté coulissant à translation.

La vitesse du berceau 210 en fonctionnement ne doit pas être trop élevée sous peine d'engendrer un décrochage des bactéries de leur support d'accrochage 220 ou d'engendrer une oxygénation de l'effluent, ni trop faible pour maintenir un rendement correct du réacteur 100. Dans la pratique, cette vitesse devra de préférence être comprise entre 5 et 50 cm/s.

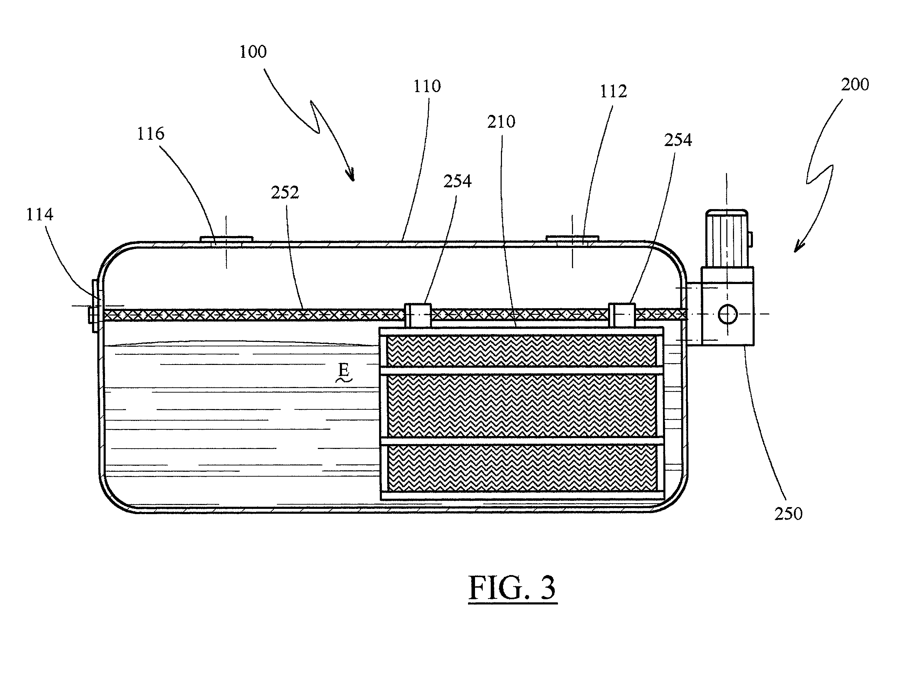

Dans une variante de réalisation représentée à la Fig. 3, le moyen d'entraînement 250 est constitué d'un motoréducteur qui peut entraîner en rotation une vis sans fin 252 traversant longitudinalement l'enveloppe 110 et autour duquel au moins un palier taraudé 254, fixé au berceau 210, est monté à mouvement hélicoïdal. Ainsi, lors de la mise en marche du motoréducteur 250, le berceau 210 se déplace suivant un mouvement de translation. La vis sans fin 252 est de préférence de type à double filet croisé permettant un mouvement de translation alternatif du berceau 210 avec un motoréducteur fonctionnant dans un seul sens de rotation. Ce type de vis sans fin 252 autorise également un déplacement du berceau 210 suivant un mouvement de translation alternatif sans utiliser de capteurs de fin de course.

L'effluent E à traiter est transféré dans le réacteur par l'orifice d'alimentation 112. Le moyen de chauffage est éventuellement mis en fonctionnement.

Les bactéries se fixent alors sur les supports d'accrochage 220. La structure en nids d'abeille de ces supports d'accrochage 220 leur permet, compte tenu de leur volume, de recevoir une quantité importante de bactéries.

Le dispositif de brassage 200 est mis en oeuvre. Le moyen d'entraînement 250 déplace suivant un mouvement de translation alternatif le berceau 210 dans le réacteur 100. Les bactéries sont alors déplacées au sein de l'effluent E et transforment l'effluent E notamment en méthane, en gaz carbonique et en un effluent résiduel pouvant être valorisé sous forme d'engrais.

Le réacteur de l'invention permet d'améliorer le rendement de traitement de l'effluent par rapport à un réacteur de l'art antérieur.

## Revendications

1. Réacteur (100) de type à axe horizontal destiné à contenir un effluent liquide (E) pour permettre le développement d'une réaction chimique de celui-ci, le réacteur (100) étant pourvu d'au moins un dispositif de brassage (200), **caractérisé en ce que** le dispositif de brassage (200) comprend un berceau (210) disposé dans le réacteur (100) et sur lequel est monté au moins un support d'accrochage (220) permettant aux bactéries de l'effluent (E) de s'y accrocher, le berceau (210) étant fixé à un moyen d'entraînement (250) permettant au berceau (210) de se déplacer dans un volume pratiquement égal à celui occupé par l'effluent (E).

2. Réacteur (100) selon la revendication 1, **caractérisé en ce que** chaque support d'accrochage (220) est constitué d'un parallélépipède formé à partir de feuilles gaufrées superposées présentant une structure en nids d'abeille.

3. Réacteur (100) selon la revendication 1 ou 2, **caractérisé en ce que** le mouvement du berceau (210) est un mouvement de translation alternatif.

4. Réacteur (100) selon la revendication 3, **caractérisé en ce que** la vitesse du berceau (210) en fonctionnement est comprise entre 5 et 50 cm/s.

5. Réacteur (100) selon l'une des revendications précédentes, **caractérisé en ce que** le berceau (210) est constitué d'un cadre fabriqué à partir de profilés métalliques (212) et dans lequel sont fixés le ou les supports d'accrochage (220).

6. Réacteur (100) selon l'une des revendications précédentes, **caractérisé en ce que** la hauteur du berceau (210) lorsqu'il est installé dans le réacteur (100) est telle qu'il demeure immergé dans l'effluent (E) contenu dans ledit réacteur 100 afin de réduire l'oxygénation de l'effluent (E) à traiter.

7. Réacteur (100) selon l'une des revendications précédentes, **caractérisé en ce que** le moyen d'entraînement (250) est un vérin à double effet.

8. Réacteur (100) selon l'une des revendications 1 à 6, **caractérisé en ce que** le moyen d'entraînement (250) est un motoréducteur destiné à entraîner en rotation une vis sans fin (252) traversant longitudinalement l'enveloppe (110) et autour duquel au moins un palier taraudé (254) est monté à mouvement hélicoïdal, le palier taraudé (254) étant fixé au berceau (210).

9. Réacteur (100) selon la revendication 8, **caractérisé en ce que** la vis sans fin (252) est de type à double filet croisé permettant un mouvement de translation alternatif du berceau (210) avec un motoréducteur fonctionnant dans un seul sens de rotation.

10. Réacteur (100) selon l'une des revendications précédentes, **caractérisé en ce que** le berceau (210) est suspendu à un dispositif de sustentation destiné à supporter sa masse afin d'éviter que le moyen d'entraînement (250) n'ait à supporter ladite masse et puisse être utilisé uniquement comme moyen de déplacement.

11. Réacteur (100) selon la revendication 10, **caractérisé en ce que** le dispositif de sustentation est constitué d'au moins un arbre (216) traversant longitudinalement l'enveloppe (110) et autour duquel au moins un palier (218) est monté coulissant à translation, le palier (218) étant fixé au berceau (210).

12. Réacteur (100) selon l'une des revendications précédentes, **caractérisé en ce que** le réacteur (100) est un réacteur de méthanisation.
